# EUROPÄISCHE PATENTSCHRIFT

(11) **EP 0 490 382 B1**
(45) Veröffentlichungstag und Bekanntmachung des Hinweises auf die Patenterteilung: **20.03.1996**
(21) Anmeldenummer: 91121361.9
(22) Anmeldetag: 12.12.1991
(51) Int. Cl.: C07C 209/48, C07C 209/70

(54) **Verfahren zur Herstellung von gesättigten primären Fettaminen durch Hydrierung von ungesättigten Fettsäurenitrilen**
Process for the preparation of saturated primary fatty amines by hydrogenation of unsaturated fatty nitriles
Procédé pour la préparation d'amines grasses primaires saturées par hydrogénation de nitriles gras insaturés

(30) Priorität: 14.12.1990 DE 4039936
(43) Veröffentlichungstag der Anmeldung: 17.06.1992
(73) Patentinhaber: HOECHST AKTIENGESELLSCHAFT, D-65926 Frankfurt am Main (DE)
(72) Erfinder: Fruth, Anton, W-8268 Garching/Alz, Hart (DE); Strauss, Julius, W-8262 Altötting (DE); Stühler, Herbert, Dr., W-8269 Burgkirchen (DE)

(56) Entgegenhaltungen:
- DE-C- 872 343
- US-A- 3 896 173
- US-A- 4 003 933
- Houben-Weyl; "Methoden der organischer Chemie", Band XI/1, Seiten 557, 566, 567

## Beschreibung

Die Erfindung betrifft ein Verfahren zur Herstellung von gesättigten primären Fettaminen durch Hydrierung von ungesättigten Fettsäurenitrilen in flüssiger Phase in Gegenwart von Nickel- oder Kobalt-Katalysatoren.

Es ist schon seit langem bekannt, primäre Fettamine aus Fettsäurenitrilen durch Hydrierung der Fettsäurenitrile in flüssiger Phase unter Verwendung von Nickel- oder Kobalt-Katalysatoren herzustellen. So wird in der US-Patentschrift 3,293,298 ein Verfahren zur Herstellung von primären Alkylaminen mit 8 bis 22 C-Atomen beschrieben, bei dem ein entsprechendes Alkylnitril in Gegenwart eines üblichen Nickel-Katalysators und in Gegenwart von Ammoniak bei einer Temperatur von 120 bis 150 °C und einem Wasserstoffdruck von 5 bis 25 bar hydriert wird. Zur Erreichung einer hohen Ausbeute an primärem Amin wird der Nickel-Katalysator in Kombination mit einer bestimmten Menge von einem festen polaren Absorbens, wie Aluminiumoxid, verwendet. Wie aus den Beispielen ersichtlich, in denen von Lauronitril, Stearonitril und Sebaconitril ausgegangen wird, werden mit diesem Verfahren gesättigte primäre Fettamine nur aus gesättigten Fettsäurenitrilen erhalten.

In der US-Patentschrift 3,574,754 wird ein Verfahren zur Herstellung von primären Fettaminen aus Fettsäurenitrilen, wie Talgfettsäurenitril, Ölsäurenitril und Cocosfettsäurenitril, beschrieben. Die Hydrierung dieser ungesättigten Fettsäurenitrile erfolgt im einzelnen in der Weise, daß die Nitrile in Gegenwart eines üblichen Nickel- oder Kobalt-Katalysators und in Gegenwart von 0,5 bis 8 mol Ammoniak, pro mol Fettsäurenitril, mit Wasserstoff bei einem Druck von etwa 30 bis 80 bar und einer Temperatur von 50 bis 200 °C in einer Stufe umgesetzt werden, wobei der Hydrierwasserstoff portionsweise zum Umsetzungsgemisch, bestehend aus Fettsäurenitril, Katalysator und eingesetztem Ammoniak, zugegeben wird. Der Wasserstoff wird vorzugsweise an verschiedenen Stellen des in einer Reaktionszone befindlichen Umsetzungsgemisches eingeführt, wobei die Einführung der ersten Wasserstoffportion nahe am Beginn der Reaktionszone erfolgt. Dieses Verfahren hat ebenfalls den Vorteil, daß die leicht zugänglichen und üblichen Nickel- oder Kobalt-Katalysatoren zur Hydrierung eingesetzt werden können. Durch die Verwendung von Ammoniak wird ferner erreicht, daß das primäre Fettamin in einer relativ hohen Ausbeute erhalten wird, die unerwünschte Bildung der Nebenprodukte, das sind sekundäres und tertiäres Fettamin, also weitgehend unterdrückt wird. Ein entscheidender Nachteil dieses Verfahrens liegt jedoch darin, daß vorhandene Doppelbindungen, wenn überhaupt, nicht vollständig zu den entsprechenden gesättigten Bindungen hydriert werden. So zeigen die Beispiele der in Rede stehenden US-Patentschrift, in denen die Hydrierung von Talgfettnitril beschrieben wird, daß das Reaktionsprodukt zwar nur geringe Mengen an sekundärem und tertiärem Amin enthält, seine Iodzahl aber sehr hoch ist (die in Tabelle I der Patentschrift für die sechs Beispiele angegebenen Iodzahlen liegen im Bereich von 38.1 bis 50.5). Aus dem eingesetzten Talgfettnitril wurde zwar ein primäres Talgfettamin erhalten, nicht aber ein primäres und gesättigtes Amin; ein solches Reaktionsprodukt würde klarerweise eine viel niedrigere Iodzahl besitzen.

Bei der Herstellung von gesättigtem primärem Fettamin durch Hydrierung von ungesättigtem Fettsäurenitril geht es also im wesentlichen darum, die Bildung von sekundärem und tertiärem Amin soweit als möglich auszuschließen und ferner die Hydrierung so zu führen, daß nicht nur die Nitrilgruppe zur primären Aminogruppe, sondern auch vorhandene Doppelbindungen im Fettsäurenitril zu gesättigten Bindungen hydriert werden. Das Reaktionsprodukt der Hydrierung von ungesättigten Fettsäurenitrilen (in der Regel liegen 1 bis 3 olefinische Bindungen in der Kohlenwasserstoffgruppe des Fettsäurenitrils vor) sollte also im wesentlichen aus dem entsprechenden primären Fettamin bestehen und gleichzeitig eine möglichst niedrige Iodzahl aufweisen, das heißt eine Iodzahl von höchstens 5, als Zeichen dafür, daß auch die Doppelbindungen im Kohlenwasserstoffrest des Fettsäurenitrils hydriert worden sind (das ungesättigte Ausgangsnitril hat in der Regel eine Iodzahl von etwa 10 bis 100).

In der US-Patentschrift 3,896,173 wird ein Verfahren zur Herstellung von gesättigten verzweigten Diaminen aus ungesättigten verzweigten Dinitrilen in zwei Hydrierstufen mit Entfernung des in der ersten Hydrierung eingesetzten Ammoniaks zwischen den beiden Hydrierstufen beschrieben. Dabei wird eine relativ große Ammoniakmenge eingesetzt und beide Hydrierungen werden bei relativ hohen Wasserstoffdrücken durchgeführt. Eine weitere Eigenart dieser Hydrierung von verzweigten Dinitrilen liegt darin, daß sie in Gegenwart eines organischen Lösungsmittels durchgeführt wird.

Aus Houben-Weyl "Methoden der organischen Chemie", Band XI/1, Seiten 557, 566 und 567 ist schließlich bekannt, daß man Nitrile mit einem Rest mit aliphatischer Doppelbindung in einem Arbeitsgang zu gesättigten Aminen hydrieren kann, wenn man die Hydrierung in Gegenwart eines Lösungsmittels oder in Gegenwart von so viel Ammoniak durchführt, daß der Ammoniak ganz oder teilweise die Rolle eines Lösungsmittels übernimmt.

Die Aufgabe der Erfindung besteht nun darin, ein Verfahren zur Hydrierung von ungesättigten Fettsäurenitrilen unter Verwendung der üblichen und leicht zugänglichen Nickel- oder Kobalt-Katalysatoren zu schaffen, mit dem primäres und gesättigtes Fettamin erhalten wird, mit dem also sowohl die olefinische(n) Bindung(en) im ungesättigten Fettsäurenitril als auch die Nitrilgruppe vollständig hydriert werden und dabei die Bildung der unerwünschten Nebenprodukte, sekundäres und tertiäres Amin, wenn überhaupt, nur in einer vernachlässigbaren Menge erfolgt. Das neue Verfahren zur Herstellung von gesättigten primären Fettaminen aus den entsprechenden ungesättigten Fettsäurenitrilen soll also primäres und gesättigtes Fettamin in sehr hoher Ausbeute und mit einer Iodzahl von höchstens 5 liefern.

Das erfindungsgemäße Verfahren zur Herstellung von gesättigten primären Fettaminen durch Hydrierung von ungesättigten Fettsäurenitrilen in flüssiger Phase in Gegenwart von Nickel- oder Kobalt-Katalysatoren ist dadurch gekennzeichnet, daß in einer ersten Reaktionsstufe die Nitrilgruppe des Fettsäurenitrils zur primären Aminogruppe hydriert wird durch Umsetzung des Fettsäurenitrils mit Wasserstoff in Gegenwart von 0,1 bis 10 Gew.-% Katalysator, bezogen auf Fettsäurenitril, und in Gegenwart von 0,5 bis 1,5 mol Ammoniak, pro mol Fettsäurenitril, bei einer Temperatur von 80 bis 160 °C und einem Druck von 10 bis 50 bar, worauf der Ammoniakgehalt im Reaktionsgemisch der Hydrierung auf einen Wert von 0,1 bis 0 mol, pro mol eingesetztes Fettsäurenitril, herabgesetzt wird, und daß in einer zweiten Reaktionsstufe die Doppelbindungen im Fettsäurenitril zu gesättigten Bindungen hydriert werden durch Umsetzung des genannten Reaktionsgemisches mit einem Ammoniakgehalt von höchstens 0,1 mol, pro mol eingesetztes Fettsäurenitril, mit Wasserstoff bei einer Temperatur von 80 bis 160 °C und einem Druck von 1 bis 25 bar, bis primäres und gesättiges Fettamin vorliegt.

Das Ausgangsmaterial für das erfindungsgemäße Verfahren sind ungesättigte Fettsäurenitrile mit 8 bis 22 C-Atomen, vorzugsweise 12 bis 18 C-Atomen, oder deren Gemische oder Gemische aus diesen Fettsäurenitrilen und aus bis zu 90 Gew.-%, vorzugsweise bis zu 60 Gew.-%, gesättigten Fettsäurenitrilen der genannten Kettenlänge, Gewichtsprozente bezogen auf die Gewichtssumme an gesättigten und ungesättigten Fettsäurenitrilen. Als Fettsäurenitrile werden also ungesättigte Fettsäurenitrile mit 8 bis 22 C-Atomen, vorzugsweise 12 bis 18 C-Atomen, und mit vorzugsweise 1 bis 3 (konjugierten oder vorzugsweise isolierten) Doppelbindungen oder deren Gemische eingesetzt, die nach bekannten Verfahren aus den entsprechenden Fettsäuren hergestellt werden können. Ebenso geeignet sind auch Gemische aus ungesättigten und gesättigten Nitrilen, vorzugsweise solche, die aus den Fettsäuren natürlich vorkommender Fette und Öle hergestellt werden, wie zum Beispiel Talgfettsäure, Cocosfettsäure, Palmkernfettsäure, Tranfettsäure, Baumwollsaatölfettsäure, Rübölsäure, Reisölsäure, Sonnenblumenölsäure und Sojaölsäure. Diese Gemische können neben den ungesättigten Fettsäurenitrilen bis zu 90 Gew.-%, vorzugsweise bis zu 60 Gew.-%, gesättigte Fettsäurenitrile mit 8 bis 22 C-Atomen, vorzugsweise mit 12 bis 18 C-Atomen, enthalten. Das erfindungsgemäße Verfahren ist also anwendbar auf ungesättigte Fettsäurenitrile mit 8 bis 22 C-Atomen, vorzugsweise 12 bis 18 C-Atomen, die 0 bis 90 Gew.-%, vorzugsweise 0 bis 60 Gew.-%, gesättigte Fettsäurenitrile mit 8 bis 22 C-Atomen, vorzugsweise 12 bis 18 C-Atomen, enthalten. Ein besonders bevorzugtes Ausgangsmaterial ist Talgfettnitril, das aus Talg tierischen Ursprungs gewonnen wird und aus etwa 40 bis 75 Gew.-% ungesättigtem C₁₆-Nitril und insbesondere C₁₈-Nitril (1 bis 3 Doppelbindungen in der Kohlenstoffkette) und etwa 25 bis 60 Gew.-% gesättigten C₁₄-, C₁₆- und C₁₈-Nitrilen zusammengesetzt ist. Die aus natürlichen Fetten stammenden Nitrilgemische können in kleinen Anteilen zusätzlich die höheren und niedrigeren geradzahligen Homologen enthalten. Die genannten Zahlen an Kohlenstoffatomen schließen den Kohlenstoff der Nitrilgruppe ein. Die in Rede stehenden Fettsäurenitrile haben Iodzahlen im Bereich von 10 bis 100. So weisen die genannten Talgfettnitrile gewöhnlich eine Iodzahl von 30 bis 60 auf (die Iodzahl gibt bekanntlich den Verbrauch g Iod pro 100 g Substanz an).

Das mit dem erfindungsgemäßen Verfahren bei Einsatz der genannten Fettsäurenitrile erhaltene Reaktionsprodukt besteht demnach aus gesättigten primären Fettaminen mit 8 bis 22 C-Atomen, vorzugsweise 12 bis 18 C-Atomen, in der Alkylkette (die Alkylketten können gleich oder verschieden sein).

Zur Durchführung des erfindungsgemäßen Verfahrens (erste Reaktionsstufe) gibt man das oder die ungesättigten Fettsäurenitrile oder das Gemisch aus ungesättigten und gesättigten Nitrilen in einen Autoklaven, der mit einem Heiz-/Kühlmantel und einem gut wirksamen Rührer ausgestattet ist. Es kann auch ein Reaktor verwendet werden, dessen Inhalt ständig umgepumpt wird. In der Umpumpleitung eingebaut ist ein Wärmetauscher mit Heiz- und Kühlmöglichkeit sowie ein Injektor, der ständig Gas aus dem Reaktor ansaugt. Die Reaktionsgefäße haben zudem Vorrichtungen zum Ein- und Ableiten von Gasen, zum Befüllen und Entleeren sowie zur Kontrolle und Regelung von Druck und Temperatur. Sofern es zweckmäßig ist, wird man das eingesetzte Fettsäurenitril in geschmolzenem Zustand vorlegen. In das Reaktionsgefäß gibt man ferner den zur Hydrierung des Nitrils vorgesehenen Katalysator. Als Katalysatoren eignen sich Nickel- und Kobalt-Katalysatoren, die mit Spuren anderer Metalle, wie zum Beispiel Calcium, Barium, Eisen, Mangan und Molybdän, dotiert sein können und die in Form von Pulverkontakten, Trägerkatalysatoren oder Raney-Katalysatoren eingesetzt werden können. Bei Pulverkontakten und Trägerkatalysatoren eignen sich als Material beispielsweise Aluminiumoxid, Silikagel, Kieselgur und Bimsstein. Bevorzugt werden Nickel-Katalysatoren verwendet, insbesondere in Form von Raney-Nickel. Die genannten Katalysatoren werden in einer Menge von 0,1 bis 10 Gew.-% zugesetzt, vorzugsweise in einer Menge von 0,5 bis 5 Gew.-%, Gewichtsprozente bezogen auf das Gewicht des eingesetzten Fettsäurenitrils (die genannten Gewichtsprozentmengen beziehen sich klarerweise auf die Elemente Nickel und Kobalt, umfassen also nicht zum Beispiel auch das Trägermaterial).

Es ist vorteilhaft, daß das Ausgangsgemisch im wesentlichen frei von Wasser ist. Die Entfernung-eventuell vorhandenen Wassers kann zum Beispiel dadurch erreicht werden, daß man das Ausgangsgemisch (Fettsäurenitril und Katalysator) auf eine Temperatur von über 100 °C erhitzt, vorzugsweise auf eine Temperatur von 120 bis 130 °C, und in bekannter Weise mit Stickstoff unter gutem Rühren spült.

Zu dem im Reaktionsgefäß befindlichen Gemisch aus Fettsäurenitril und Katalysator wird noch Ammoniak zugesetzt (flüssig), und zwar in einer Menge von 0,5 bis 1,5 mol, pro mol eingesetztes Fettsäurenitril. Die Hydrierung in der ersten Stufe des erfindungsgemäßen Verfahrens, das heißt die Hydrierung der Nitrilgruppe, erfolgt in Gegenwart der angegebenen Ammoniakmenge bei einem Druck (Gesamtdruck) von 10 bis 50 bar, vorzugsweise 20 bis 40 bar, und einer Temperatur von 80 bis 160 °C, vorzugsweise von 100 bis 140 °C. Von den angegebenen Druck- und Temperaturwerten wird man die höheren dann wählen, wenn man eine relativ kurze Reaktionszeit erreichen möchte. Mit der Zufuhr von Hydrierwasserstoff in das Reaktionsgefäß kann vor oder nach dem Erhitzen auf die Reaktionstemperatur begonnen werden (es ist zweckmäßig die erste Wasserstoffmenge vor dem Erhitzen zuzusetzen). Der Hydrierwasserstoff kann kontinuierlich oder portionsweise unter Aufrechterhalten der angegebenen Temperatur und des angegebenen Druckes in das Reaktionsgefäß eingebracht werden, wobei klarerweise für einen innigen Kontakt mit dem Reaktionsgemisch gesorgt wird (zum Beispiel durch kräftiges Rühren, ständiges Umpumpen oder Kreisgasfahrweise). Der Hydrierwasserstoff wird in einer solchen Menge und solange aufgedruckt, bis im wesentlichen alle -C≡N-Gruppen hydriert sind. Dies ist dann der Fall, wenn nur noch höchstens 2 Gew.-% vom eingesetzten Nitril, vorzugsweise höchstens 0,5 Gew.-%, feststellbar sind. Die -C≡N-Gruppen sind im wesentlichen zu primären Aminogruppen hydriert worden. Auch olefinische Doppelbindungen können in der ersten Stufe hydriert worden sein (diese Hydrierung umfaßt - wie eine Bestimmung der Iodzahl zeigt - in der Regel höchstens bis zu 20 Mol-% der insgesamt vorhandenen Doppelbindungen). Entscheidend ist jedenfalls, daß nach dem Hydriervorgang der ersten Reaktionsstufe im Reaktionsgemisch im wesentlichen keine Nitrilgruppen mehr vorliegen. Die Zeit für diese erste Hydrierung umfaßt in der Regel 2 bis 4 Stunden und hängt im wesentlichen vom Katalysator und der Reaktionstemperatur ab.

Nach Abschluß der Hydrierung in der ersten Stufe des erfindungsgemäßen Verfahrens wird das anwesende Ammoniak bis auf einen Wert von höchstens 0,1 mol, vorzugsweise höchstens 0,05 mol, pro mol eingesetztes Fettsäurenitril, entfernt. Die Entfernung des Ammoniaks aus dem Reaktionsgemisch (und damit auch aus dem Gasraum des Reaktionsgefäßes), anschließend an die erste Hydrierung, kann beispielsweise einfach durch Entspannen erreicht werden. Die Einstellung der angegebenen Ammoniakwerte kann zum Beispiel auch durch Auswaschen mit Wasser oder mit Hilfe anderer Absorptionsmittel durchgeführt werden. Die Temperatur, die bei diesem Prozeß vorliegt, ist an sich nicht kritisch und kann den Gegebenheiten angepaßt werden. So wird man das genannte Entspannen bei einer Temperatur von etwa 80 bis 130 °C vornehmen. Ein starkes Abkühlen des Reaktionsgemisches wird man schon deswegen vermeiden, weil dann das Reaktionsgemisch für die weitere Hydrierung unter Umständen wieder aufzuheizen ist. Es ist bevorzugt, das nach der ersten Hydrierung vorliegende Reaktionsgemisch vom anwesenden Ammoniak praktisch vollständig zu befreien. Die angewandte Arbeitsweise zur Entfernung des Ammoniaks (Auswaschen durch Kreisgasführung, Entspannen und dergleichen) ist nicht kritisch.

Nach Einstellen der genannten Ammoniakwerte von 0 bis 0,1 mol, vorzugsweise 0 bis 0,05 mol, pro mol eingesetztes Fettsäurenitril, im Reaktionsgemisch nach der ersten Hydrierung wird die zweite Hydrierung durchgeführt, das ist die Hydrierung der im Reaktionsgemisch noch vorhandenen olefinischen Doppelbindungen. Diese Hydrierung wird bei einem Druck von 1 bis 25 bar, und einer Temperatur von 80 bis 160 °C, vorzugsweise 100 bis 140 °C, durchgeführt. Falls das Reaktionsgemisch nach der ersten Hydrierung abgekühlt worden ist, ist es nun wieder auf Reaktionstemperatur zu erhitzen. Die zweite Hydrierung wird in der Regel bei etwa der gleichen Temperatur wie die erste Hydrierung durchgeführt. Der Druck bei der zweiten Hydrierung kann im Vergleich zur ersten Hydrierung wesentlich niedriger sein. So kann die zweite Hydrierung auch bei Normaldruck (1 bar), das heißt drucklos, durchgeführt werden. In diesem Fall sollte eventuelles Eindringen von Luft in das Reaktionsgefäß durch entsprechende Maßnahmen, zum Beispiel geringem Überdruck, verhindert werden. Das entscheidene Merkmal der zweiten Hydrierung im Vergleich zur ersten Hydrierung liegt aber, wie bereits erwähnt, darin, daß kein oder nur sehr wenig Ammoniak anwesend ist. Was die Katalysatormenge für die zweite Hydrierung betrifft, so genügt in der Regel die zu Beginn eingesetzte Katalysatormenge, auch wenn damit bereits eine Hydrierung durchgeführt worden ist. Die Hydrierung selbst kann analog der ersten Hydrierung durchgeführt werden. Nachdem die gewünschte Reaktionstemperatur vorliegt, wird also der Hydrierwasserstoff zugeführt. Er kann kontinuierlich oder portionsweise unter Aufrechterhalten der Reaktionstemperatur eingebracht werden. Der Hydrierwasserstoff wird in einer solchen Menge und solange zugeführt, bis keine oder praktisch keine Doppelbindung mehr feststellbar ist (Iodzahlbestimmung). Die Zeit für die zweite Hydrierung liegt im Bereich von vorzugsweise 1 bis 4 Stunden. Sie hängt ebenso wie die erste Hydrierung vom Katalysator und der Reaktionstemperatur ab. In der Regel wird man in der ersten Reaktionsstufe bereits so viel Katalysator einsetzen, daß auch die zweite Hydrierung in der angegebenen Zeit abgeschlossen ist. Da die Zeit für die zweite Hydrierung aber nicht nur vom Katalysator und der Temperatur abhängt, sondern auch von der Menge an eventuell vorhandenem Ammoniak (ein höherer Ammoniakgehalt bedingt bei gleichem Katalysator und gleicher Reaktionstemperatur eine längere Reaktionszeit), wird man in der zweiten Stufe frischen Katalysator zusetzen, wenn man in der ersten Stufe relativ wenig Katalysator eingesetzt hat. Nach der zweiten Hydrierung liegt das angestrebte primäre und gesättigte Fettamin vor.

Falls die Abtrennung des primären gesättigten Fettamins vom eingesetzten Katalysator gewünscht wird, kann dies zum Beispiel einfach durch Dekantierung oder Filtrierung erreicht werden. Der zurückgewonnene Katalysator ist auch für weitere erfindungsgemäße Hydrierungen geeignet.

Das erfindungsgemäße Verfahren weist eine Reihe von Vorteilen auf. Es ist einfach in der Durchführung. Es liefert die angestrebten primären gesättigten Fettamine mit einer Iodzahl von weniger als 5 und in einer hohen Ausbeute, das heißt ohne eine nennenswerte Menge an den Nebenprodukten sekundäres und tertiäres Fettamin. Die hohe Ausbeute und Reinheit des Fettamins wird in einer relativ kurzen Gesamtreaktionszeit erreicht. Das erfindungsgemäße Verfahren zeichnet sich also auch durch eine hohe Wirtschaftlichkeit aus. Das erhaltene primäre gesättigte Fettamin ist ferner von heller Farbe und behält die gute Farbe zum Beispiel auch im Falle von Alkoxylierungsreaktionen bei. Die in Rede stehenden primären gesättigten Fettamine sind bekanntlich wertvolle Produkte zur Herstellung von Waschmitteln, Herbiziden, Desinfektionsmitteln, Antistatika, Antibackmittel, Textilausrüstungsmitteln und Flotationsmittel.

Die Erfindung wird nun an Beispielen noch näher erläutert.

### Beispiel 1

In einem 2-l-Rührautoklaven werden 800 g (3,2 mol) Ölsäurenitril mit Iodzahl 88 und 40 g Raney-Nickel (das sind 5,0 Gew.-% Nickel, bezogen auf Ölsäurenitril) vorgelegt. Nach Spülung mit Stickstoff werden 70 g (4,1 mol) flüssiger Ammoniak zugegeben (das sind 1,3 mol NH₃ pro mol Ölsäurenitril). Die Mischung wird unter Rühren auf 130 °C erhitzt. Bei dieser Temperatur wird Wasserstoff bis auf einen Druck von 35 bar aufgedrückt, und dieser Druck wird durch weitere Zufuhr von Wasserstoff über eine Druckregelung aufrechterhalten. Nach 2 ½ Stunden Reaktionszeit wird kein Wasserstoff mehr aufgenommen, was das Ende der Hydrierung der Nitrilgruppe des Ölsäurenitrils anzeigt. Nun wird der Rührautoklav vollständig entspannt und der auf 60 °C abgekühlte Autoklaveninhalt in einen offenen 2-l-Rührkolben übergeführt. Das erhaltene primäre Oleylamin hat die folgenden Analysenwerte:

| | |
|---|---|
| primärer Stickstoff in Äquivalentprozent | 96,7 |
| nicht primärer Stickstoff in Äquivalentprozent | 3,3 |
| Restgehalt an Ölsäurenitril in Gewichtsprozent | 0,3 |
| Iodzahl | 73 |

Durch das genannte Entspannen und Überführen des Reaktionsgemisches (inclusive Katalysator) in den 2-l-Rührkolben beträgt der Ammoniakgehalt im Reaktionsgemisch nur noch 1 g (0,06 mol), das sind 0,019 mol NH₃ pro mol eingesetztes Ölsäurenitril. Zur Durchführung der zweiten Hydrierung (zweite Reaktionsstufe) wird der Inhalt des offenen Rührkolbens zunächst mit Stickstoff gespült und dann auf 140 °C erhitzt und bei dieser Temperatur gehalten. Bereits während des Erhitzens wird mit der Zufuhr von Wasserstoff begonnen, und zwar in einer Menge von 55 l Wasserstoff pro Stunde. Diese Wasserstoffzufuhr wird 3 ½ Stunden lang fortgeführt. Nach dieser Zeit ist die bei Normaldruck durchgeführte Hydrierung der Doppelbindungen im Oleylrest des primären Oleylamins beendet. Es liegt ein gesättigtes primäres Amin (Stearylamin) mit den folgenden Analysenwerten vor:

| | |
|---|---|
| primärer Stickstoff in Äquivalentprozent | 96,0 |
| nicht primärer Stickstoff in Äquivalentprozent | 4,0 |
| Restgehalt an Ölsäurenitril in Gewichtsprozent | <0,1 |
| Iodzahl | 4 |

### Beispiel 2

700 g (3,5 mol) Cocosfettsäurenitril mit einer Iodzahl von 10 und 30 g Raney-Nickel, abfiltriert aus dem Reaktionsgemisch nach der zweiten Hydrierung im Beispiel 1 (das sind 4,3 Gew.-% Nickel, bezogen auf Cocosfettsäurenitril), werden in einem 2-l-Rührautoklaven vorgelegt. Nach Spülung mit Stickstoff und Zugabe von 90 g (5,3 mol) flüssigem Ammoniak (das sind 1,5 mol NH₃ pro mol Cocosfettsäurenitril) wird Wasserstoff bis auf einen Druck von 30 bar aufgedrückt, worauf die Mischung unter Rühren auf 135 °C erhitzt wird. Bei dieser Temperatur wird so lange Wasserstoff bis auf einen Druck von maximal 40 bar immer wieder nachgedrückt, bis kein Druckabfall mehr festzustellen ist. Dies ist nach 3 ¼ Stunden der Fall. Das gebildete primäre Cocosfettamin hat die folgenden Analysenwerte:

| | |
|---|---|
| primärer Stickstoff in Äquivalentprozent | 97,1 |
| nicht primärer Stickstoff in Äquivalentprozent | 2,9 |
| Restgehalt an Cocosfettsäurenitril in Gewichtsprozent | 0,1 |
| Iodzahl | 9 |

Zur Entfernung von Ammoniak aus dem Autoklaven und aus dem Reaktionsgemisch der ersten Hydrierung wird der Autoklav völlig entspannt und sein Inhalt bei etwa 80 °C so lange mit Stickstoff gespült, bis der Ammoniakgehalt im Reaktionsgemisch nur noch 0,5 g (0,03 mol) beträgt (das sind 0,009 mol NH₃ pro mol eingesetztes Cocosfettsäurenitril). Nun wird der Autoklav mit Wasserstoff stickstofffrei gespült und dann wird Wasserstoff bis auf einen Druck von 6 bar aufgedrückt. Nach dem Einschalten des Rührwerks und Erhitzen der Mischung auf 135 °C erfolgt die Hydrierung der Doppelbindungen, indem der verbrauchte Wasserstoff durch eine Druckregelung ständig nachgeführt und der genannte Druck von 6 bar aufrechterhalten wird. Nach 3 ½ Stunden ist die Reaktion beendet. Das erhaltene gesättigte primäre Amin hat folgende Analysenwerte:

| | |
|---|---|
| primärer Stickstoff in Äquivalentprozent | 96,5 |
| nicht primärer Stickstoff in Äquivalentprozent | 3,5 |
| Restgehalt an Cocosfettsäurenitril in Gewichtsprozent | <0,1 |
| Iodzahl | 3 |

### Beispiel 3

35 kg (140 mol) Talgfettnitril werden zusammen mit 0,88 kg Raney-Nickel (das sind 2,5 Gew.-% Nickel, bezogen auf Talgfettnitril) und 1,75 kg (102,9 mol) Ammoniak (das sind 0,74 mol NH₃ pro mol Talgfettnitril) in einen vorher mit Stickstoff gespülten Reaktor eingelegt. Der Reaktor ist so ausgestattet, daß sein Inhalt mit einer Pumpe über einen Wärmetauscher und einen Injektor zurück in den Reaktor gepumpt werden kann. Der Injektor saugt dabei gleichzeitig Gas aus dem Reaktor an und mischt es intensiv mit der Flüssigkeit. Das Talgfettnitril besteht aus 65 Gew.-% Fettsäurenitril mit 18 C-Atomen und 35 Gew.-% Fettsäurenitril mit im wesentlichen 14 und 16 C-Atomen, wobei 50 Gew.-% ungesättigtes Fettsäurenitril mit 1 bis 3 Doppelbindungen und 50 Gew.-% gesättigtes Fettsäurenitril sind; die Iodzahl ist 57.

Nach dem Einschalten der Reaktorpumpe wird die Reaktionsmischung auf 125 °C erhitzt. Es stellt sich ein Druck von 22 bar ein. Nun wird dem System ständig Hydrierwasserstoff zugeführt, wobei 28 bar und 130 °C nicht überschritten werden (die Reaktionswärme wird durch den erwähnten Wärmetauscher in der Produktschleife abgeführt). Nach 3 Stunden ist die Wasserstoffaufnahme beendet. Es hat sich ein primäres Talgfettamin mit den folgenden Kennzahlen gebildet:

| | |
|---|---|
| primärer Stickstoff in Äquivalentprozent | 97,2 |
| nicht primärer Stickstoff in Äquivalentprozent | 2,8 |
| Restgehalt an Talgfettnitril in Gewichtsprozent | 0,2 |
| Iodzahl | 45 |

Zur Ammoniak-Entfernung wird nun der Reaktor bei laufender Reaktorpumpe und der Temperatur von 130 °C auf 2 bar entspannt, wodurch der Ammoniakgehalt im System (in der Reaktionsmischung) auf 0,13 kg (7,6 mol) reduziert wird (das sind 0,05 mol NH₃ pro mol eingesetztes Talgfettnitril). Die auf 130 °C gehaltene Reaktionsmischung wird nun wieder mit Wasserstoff bis auf einen Druck von 25 bar beaufschlagt und durch ständige Wasserstoffzugabe bei diesem Druck gehalten (das ist die zweite Reaktionsstufe oder die zweite Hydrierung). Nach 3 Stunden ist die Hydrierung der Doppelbindungen abgeschlossen. Es liegt ein gehärtetes Talgfettamin mit folgenden Kennzahlen vor:

| | |
|---|---|
| primärer Stickstoff in Äquivalentprozent | 96,6 |
| nicht primärer Stickstoff in Äquivalentprozent | 3,4 |
| Restgehalt an Talgfettnitril in Gewichtsprozent | <0,1 |
| Iodzahl | 4 |

### Beispiel 4

30 kg (120 mol) Talgfettnitril vom Beispiel 3 werden im mit Stickstoff inertisierten Reaktor des Beispiels 3 eingelegt, zusammen mit 0,6 kg Nickel-Trägerkatalysator, der aus 50 Gew.-% Nickel und 50 Gew.-% Siliciumdioxid als Trägermaterial besteht (das sind 1,0 Gew.-% Nickel, bezogen auf Talgfettnitril) und 2 kg (117,6 mol) Ammoniak (das sind 0,98 mol NH₃ pro mol Talgfettnitril). Der Nickel-Katalysator war bereits dreimal in der beschriebenen Zwei-Stufen-Reaktion im Einsatz.

Nach dem Einschalten der Reaktorpumpe wird die Reaktionsmischung auf 120 °C erhitzt, worauf Wasserstoff bis auf einen Druck von 30 bar aufgedrückt wird. Die Zufuhr von Hydrierwasserstoff wird fortgesetzt, wobei 30 bar und 125 °C nicht überschritten werden. Nach 2,5 Stunden ist die Wasserstoffaufnahme beendet. Es hat sich primäres Talgfettamin mit den folgenden Kennzahlen gebildet:

| | |
|---|---|
| primärer Stickstoff in Äquivalentprozent | 98,2 |
| nicht primärer Stickstoff in Äquivalentprozent | 1,8 |
| Restgehalt an Talgfettnitril in Gewichtsprozent | 0,2 |
| Iodzahl | 48,0 |

Die Entfernung des Ammoniaks aus dem System erfolgt in der Weise, daß die Gasphase durch einen mit Wasser betriebenen Adsorber geleitet wird. Die Adsorption des Ammoniaks ist nach 15 Minuten beendet. Der Reaktorinhalt hat sich während dieser Zeit auf 95 °C abgekühlt und sein Ammoniakgehalt beträgt nur noch 0,06 kg (3,5 mol, das sind 0,03 mol NH₃ pro mol eingesetztes Talgfettnitril). Nun wird der Wäscher außer Betrieb gesetzt und der Reaktorinhalt zur zweiten Hydrierung auf 125 °C erhitzt und bei dieser Temperatur so lange mit Wasserstoff beaufschlagt, bis der Druck bei 18 bar konstant bleibt, was nach 2,5 Stunden der Fall ist. Es liegt ein gehärtetes Talgfettamin mit den folgenden Werten vor:

| | |
|---|---|
| primärer Stickstoff in Äquivalentprozent | 97,5 |
| nicht primärer Stickstoff in Äquivalentprozent | 2,5 |
| Restgehalt an Talgfettnitril in Gewichtsprozent | <0,1 |
| Iodzahl | 3 |

## Patentansprüche

1. Verfahren zur Herstellung von gesättigten primären Fettaminen durch Hydrierung von ungesättigten Fettsäurenitrilen in flüssiger Phase in Gegenwart von Nickel- oder Kobalt-Katalysatoren, dadurch gekennzeichnet, daß in einer ersten Reaktionsstufe die Nitrilgruppe des Fettsäurenitrils zur primären Aminogruppe hydriert wird durch Umsetzung des Fettsäurenitrils mit Wasserstoff in Gegenwart von 0,1 bis 10 Gew.-% Katalysator, bezogen auf Fettsäurenitril, und in Gegenwart von 0,5 bis 1,5 mol Ammoniak, pro mol Fettsäurenitril, bei einer Temperatur von 80 bis 160 °C und einem Druck von 10 bis 50 bar, worauf der Ammoniakgehalt im Reaktionsgemisch der Hydrierung auf einen Wert von 0,1 bis 0 mol, pro mol eingesetztes Fettsäurenitril, herabgesetzt wird, und daß in einer zweiten Reaktionsstufe die Doppelbindungen im Fettsäurenitril zu gesättigten Bindungen hydriert werden durch Umsetzung des genannten Reaktionsgemisches mit einem Ammoniakgehalt von höchstens 0,1 mol, pro mol eingesetztes Fettsäurenitril, mit Wasserstoff bei einer Temperatur von 80 bis 160 °C und einem Druck von 1 bis 25 bar, bis primäres und gesättiges Fettamin vorliegt.

2. Verfahren nach Anspruch 1, wobei die Hydrierung in der ersten Reaktionsstufe in Gegenwart von 0,5 bis 5 Gew.-% Katalysator, bezogen auf Fettsäurenitril, und bei einer Temperatur von 100 bis 140 °C und einem Druck von 20 bis 40 bar durchgeführt wird, worauf der Ammoniakgehalt im Reaktionsgemisch der Hydrierung auf einen Wert von 0,05 bis 0 mol, pro mol eingesetztes Fettsäurenitril, herabgesetzt wird, und in der zweiten Reaktionsstufe das genannte Reaktionsgemisch mit einem Ammoniakgehalt von höchstens 0,05 mol, pro mol eingesetztes Fettsäurenitril, bei einer Temperatur von 100 bis 140 °C hydriert wird, bis primäres und gesättiges Fettamin vorliegt.

3. Verfahren nach Anspruch 1 oder 2, wobei als Katalysator Nickelkatalysatoren eingesetzt werden.

4. Verfahren nach einem der Ansprüche 1 bis 3, wobei als ungesättigte Fettsäurenitrile solche mit 8 bis 22 C-Atomen eingesetzt werden.

5. Verfahren nach einem der Ansprüche 1 bis 3, wobei als ungesättigte Fettsäurenitrile Gemische aus ungesättigten Fettsäurenitrilen mit 8 bis 22 C-Atomen und aus bis zu 90 Gew.-% gesättigten Fettsäurenitrilen mit 8 bis 22 C-Atomen eingesetzt werden, Gewichtsprozente bezogen auf die Gewichtssumme aus ungesättigtem und gesättigtem Fettsäurenitril.

## Claims

1. A process for the preparation of saturated primary fatty amines by hydrogenation of unsaturated fatty acid nitriles in the liquid phase in the presence of nickel catalysts or cobalt catalysts, which comprises a first reaction step in which the nitrile group of the fatty acid nitrile is hydrogenated to the primary amino group by reaction of the fatty acid nitrile with hydrogen in the presence of 0.1 to 10% by weight of the catalyst, relative to the fatty acid nitrile, and in the presence of 0.5 to 1.5 mol of ammonia per mole of fatty acid nitrile, at a temperature of 80 to 160°C and a pressure of 10 to 50 bar, after which the ammonia content in the hydrogenation reaction mixture is reduced to a value of 0.1 to 0 mol per mole of fatty acid nitrile used, and a second reaction step in which the double bonds in the fatty acid nitrile are hydrogenated to saturated bonds by reaction of said reaction mixture having an ammonia content of at most 0.1 mol per mole of fatty acid nitrile used, with hydrogen at a temperature of 80 to 160°C and a pressure of 1 to 25 bar.

2. The process as claimed in claim 1, wherein the hydrogenation in the first reaction step is carried out in the presence of 0.5 to 5% by weight of the catalyst, relative to the fatty acid nitrile, and at a temperature of 100 to 140°C, and a pressure of 20 to 40 bar, after which the ammonia content in the hydrogenation reaction mixture is reduced to a value of 0.05 to 0 mol per mole of fatty acid nitrile used, and in the second reaction step said reaction mixture having an ammonia content of at most 0.05 mol per mole of fatty acid nitrile used, is hydrogenated at a temperature of 100 to 140°C.

3. The process as claimed in claim 1 or 2, wherein nickel catalysts are used as the catalyst.

4. The process as claimed in any one of claims 1 to 3, wherein the unsaturated fatty acid nitriles used are those having 8 to 22 carbon atoms.

5. The process as claimed in any one of claims 1 to 3, wherein the unsaturated fatty acid nitriles used are mixtures of unsaturated fatty acid nitriles having 8 to 22 carbon atoms and up to 90% by weight of saturated fatty acid nitriles having 8 to 22 carbon atoms, percentages by weight being based on the sum of the weights of the unsaturated and saturated fatty acid nitrile.

## Revendications

1. Procédé de préparation d'amines grasses primaires , saturées, par hydrogénation de nitriles d'acides gras insaturés en phase liquide en présence de catalyseurs au nickel ou au cobalt caractérisé en ce que, dans une première étape de réaction, on hydrogène le groupe nitrile du nitrile d'acide gras en le groupe amino primaire par réaction du nitrile d'acide gras avec de l'hydrogène en présence de 0,1 à 10 % en poids d'un catalyseur par rapport au nitrile d'acide gras et en présence de 0,5 à 1,5 moles d'ammoniac par mole de nitrile d'acide gras, à une température de 80 à 160°C et sous une pression de 10 à 50 bar, ce après quoi on abaisse la teneur en ammoniac du mélange réactionnel d'hydrogénation à une valeur de 0,1 à 0 mole par mole de nitrile d'acide gras utilisé et en ce que, dans une deuxième étape de la réaction, on hydrogène les doubles liaisons du nitrile d'acide gras en liaisons saturées par réaction du mélange réactionnel ci-dessus, ayant une teneur en ammoniac au plus égale à 0,1 mole par mole de nitrile d'acide gras utilisé, avec de l'hydrogène à une température de 80 à 160°C et sous une pression de 1 à 25 bar, jusqu'à ce que soit présente une amine grasse primaire et saturée.

2. Procédé selon la revendication 1, dans lequel l'hydrogénation de la première étape de réaction est mise en oeuvre en présence de 0,5 à 5 % en poids de catalyseur, par rapport au nitrile d'acide gras et à une température de 100 à 140°C et sous une pression de 20 à 40 bar, la teneur du mélange réactionnel de l'hydrogénation en ammoniac étant ainsi abaissée à une valeur de 0,05 à 0 mole par mole de nitrile d'acide gras utilisé et, dans la deuxième étape, le mélange réactionnel mentionné ci-dessus, ayant une teneur en ammoniac au plus égale à 0,05 mole par mole de nitrile d'acide gras utilisé, est hydrogéné à une température de 100 à 140°C jusqu'à ce qu'on soit en présence d'une amine grasse primaire et saturée.

3. Procédé selon la revendication 1 ou 2, dans lequel on utilise comme catalyseur des catalyseurs au nickel.

4. Procédé selon l'une des revendications 1 à 3, dans lequel on utilise comme nitriles d'acides gras insaturés ceux qui ont de 8 à 22 atomes de carbone.

5. Procédé selon l'une des revendications 1 à 3, dans lequel on utilise comme nitriles d'acides gras insaturés des mélanges de nitriles d'acides gras insaturés ayant de 8 à 22 atomes de carbone et d'une quantité allant jusqu'à 90 % en poids de nitriles d'acides gras saturés ayant de 8 à 22 atomes de carbone, les pourcentages en poids étant rapportés au poids total du nitrile d'acide gras et du nitrile d'acide gras saturé.
